(19) Europäisches Patentamt
European Patent Office
Office européen des brevets

(11) EP 1 025 860 B1

(12) EUROPÄISCHE PATENTSCHRIFT

(45) Veröffentlichungstag und Bekanntmachung des Hinweises auf die Patenterteilung:
**12.01.2005 Patentblatt 2005/02**

(51) Int Cl.[7]: **A61K 47/48**, A61K 38/44, A61K 38/48, A61K 38/47, A61K 38/46, A61K 38/38, A61P 17/02

(21) Anmeldenummer: **00100556.0**

(22) Anmeldetag: **12.01.2000**

(54) **Proteinhaltige Hydrogele**

Protein-containing hydrogels

Hydrogels protéiniques

(84) Benannte Vertragsstaaten:
**AT DE ES FR GB IT NL SE**

(30) Priorität: **29.01.1999 DE 19903665**

(43) Veröffentlichungstag der Anmeldung:
**09.08.2000 Patentblatt 2000/32**

(73) Patentinhaber: **Beiersdorf Aktiengesellschaft**
**20245 Hamburg (DE)**

(72) Erfinder:
- **Ettner, Norbert, Dr.**
  **86551 Aichach (DE)**
- **Schink, Michael, Dr.**
  **22605 Hamburg (DE)**
- **Schreiber, Jörg, Dr.**
  **22087 Hamburg (DE)**
- **Meier, Wolfgang, Dr.**
  **4053 Basel (CH)**
- **Sauer, Marc**
  **29221 Celle (DE)**

(56) Entgegenhaltungen:
**WO-A-91/02763** **WO-A-92/00748**

- **ZALIPSKY S: "FUNCTIONALIZED POLY(ETHYLENE GLYCOL) FOR PREPARATION OF BIOLOGICALLY RELEVANT CONJUGATES" BIOCONJUGATE CHEMISTRY, AMERICAN CHEMICAL SOCIETY, WASHINGTON, US, Bd. 6, Nr. 2, 1995, Seiten 150-165, XP002068523 ISSN: 1043-1802**

EP 1 025 860 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Seit Menschengedenken ist die Heilung von therapieresistenten Wunden eine große Herausforderung für die Medizin und Naturwissenschaften. Die heutigen Anforderungen an die Funktion von interaktiven Wundauflagen für chronische Wunden gehen zurück auf Winter (1962, Nature 193, 293) und sind jüngst von Turner neu formuliert (1994, Wound Rep. Reg. 2, 202). Im Vordergrund steht dabei die Schaffung eines feuchten Wundheilungsmilieus, das im Gegensatz zur traditionellen trockenen Wundbehandlung mit zum Beispiel Mullkompressen den natürlichen Abläufen der Wundheilung physiologische und damit bessere Konditionen bietet.

Das Prinzip der feuchten Wundheilung kann derzeit als der Stand der Technik in der Therapie schwer oder nicht heilender Wunden angesehen werden. Die Wundauflage muß die Hauptmenge des Exsudates aufnehmen und gleichzeitig aber auf der Wunde selbst einen Flüssigkeitsfilm belassen, in dem die eigentliche feuchte Wundheilung stattfindet. Bei trockenen und schwachexudierenden Wunden muß die Versorgung der Wunde mit ausreichend Feuchtigkeit erfolgen, um eine Rehydrierung des dehydratisierten Gewebes zu erreichen. In der auf diese Weise etablierten feuchten Wunde kommt es dann zur Proliferation von neuen Blutgefäßen und verminderten Bakterienwachstum unter Einstellung eines geeigneten pH-Wertes.
Erfüllt werden diese Anforderungen von schwammartigen Strukturen, wie beispielsweise Hydrogele, die über einen Überschuß in Form von gebundenem Wasser verfügen.

Hydrogele werden im allgemeinen dadurch hergestellt, daß hydrophile, kohärent vorliegende Monomere in einem Dispersionsmittel eine dreidimensionale Raumnetzstruktur ausbilden, in dessen Zwischenräumen sich das Dispersionsmittel, üblicherweise Wasser, einlagern kann. Ein Hydrogel sollte über eine gewisse Sauerstoffdurchlässigkeit verfügen und eine Barrierefunktion gegenüber möglicherweise aus der Umgebung eindringende Keime einnehmen. Eine grundlegende Anforderung an ein Hydrogel sind auch ein einfacher Herstellungsprozeß sowie eine gewisse Lagerstabilität.

Der Gebrauch und die Vielfältigkeit der möglichen Anwendungen von Hydrogelen in der Wundbehandlung, sowie deren Zusammensetzung und Herstellung ist hinreichend dokumentiert (Peppas in: Hydrogels in Medicine and Pharmacy, 1986, CRC Press, Volume II, Chapter 4). Hydrogele werden bevorzugt für die Behandlung von trockenen nekrotischen Wunden wie zum Beispiel Brandwunden und chronisch venöse Ulcera eingesetzt (Thomas, in: Wound Management and Dressings, 1990, The Pharmaceutical Press, London, S. 50).
Sie bestehen aus unlöslichen polymeren Materialien, die in der Lage sind, in wäßrigen Medien aufzuquellen. Des weiteren sollten sie über einen hohen Wassergehalt verfügen, inert sein gegen biologische Prozesse, permeabel für zelluläre Metaboliten und vor allem im Kontakt mit lebendem Gewebe keine Reizungen auslösen.

Die Synthese eines biokünstlichen oder semi-synthetischen Hydrogels kann dadurch erzielt werden, daß das hydrophile synthetische Monomer kovalent an die Oberfläche eines Proteins verknüpft wird, wobei es dann innerhalb des Dispersionsmittels zur Ausbildung einer dreidimensionalen Polymer-Proteinmatrix kommt. Diese Klasse von Hydrogelen, bestehend aus synthetischen Polymeren und Biopolymeren, ist seit kurzer Zeit Gegenstand der Forschung. Diese neuartigen Biomaterialien werden als biokünstliche Hydrogele bezeichnet (Giusti et al., 1993, Trends in Polymeric Science, 9, 261).

In US 5,804,213 wird die Herstellung eines biologisch aktiven, wasserhaltigen Gels als Wundauflage berichtet. Dabei wird ein trockenes, hydrokolloidales Polymer mit Wasser und einer biologisch aktiven Substanz vermischt.

Die Verwendung von dehydratisierten, vemetzten Collagenmaterialien als Drug-Delivery-System wird in WO 98/22153 beschrieben. In WO 96/31551 werden im trockenen Zustand Proteine oder Peptide als aktive Agentien zu Polyurethan-vernetzten Microgelen gemischt. Die Microgele quellen im wäßrigen Medium zu Hydrogelen auf und setzen aus der Hydrogelmatrix das Protein bzw. das Peptid wieder frei. Auch US 5,000,955 beschreibt Polyurethan-Hydrogele für kosmetische, biologische und medizinische Anwendungen.
Kubische Phasen bestehend aus Glycerylmonooleat können Enzyme durch nichtkovalente Bindungen, wie in WO 96/39125 berichtet, immobilisieren. Dabei bleibt durch die Immobilisierung die enzymatische Aktivität erhalten und ist sogar, im Vergleich zu gelöstem Enzym, über einen längeren Zeitraum erhöht. Hervorzuheben ist weiterhin die Barrierefunktion der Gelmatrix, die den proteolytischen Abbau der immobilisierten Enzyme, wie in der Wundflüssigkeit anzutreffen, unterbindet.

Von besonderem Interesse sind Hydrogele, in die ein Biomolekül kovalent eingebunden ist, wie in US 5,733,563 und 1994 von Fortier (1994, Biotechnol.Techn., 8, 71) beschrieben. Diese Hydrogele werden durch Copolymerisation von einem durch 4-Nitrophenylchloroformiat aktiviertem Polyethylenglykol und bovinem Serumalbumin in Boratpuffer gebildet. Dabei kann die aktivierte Gruppe mit einer Amino-, SH-, OHoder COOH-Gruppe des Proteins reagieren.

Nachteile der im Stand der Technik bekannten biokünstlichen Hydrogele sind die hohe Gelbildungszeiten von 20 bis zu 270 Minuten, die Entstehung von Spaltprodukten in Form von p-Nitrophenolen in der Hydrogelmatrix und die fehlende Möglichkeit oligomere Proteine wie zum Beispiel dimere SOD oder tetramere Katalase stabil mit dem 4-Nitrophenylchloroformiat aktivierten Polyethylenglykol zu vernetzen.

Aufgabe der Erfindung ist es, wasserunlösliche, wasserquellbare Hydrogele mit kurzen Gelbildungszeiten zu fertigen, die für medizinische Zwecke einsetzbar sind und sich in besonderem Maße zur Förderung der Heilung von chronischen Wunden eignen.

Gelöst wird diese Aufgabe durch ein Hydrogel, das aus zumindest einem Protein und/oder Enzym und PEGs besteht, wobei die Verbindung zwischen den Proteine und/oder Enzymen und den PEGs über Harnstoffgruppen erfolgt. Bei der Herstellung des Hydrogels werden zusätzliche Substanzen verwendet, die mit den in Wundflüssigkeiten von chronischen Wunden vorhandenen Sauerstoffspezies (ROS) wechselwirken, d.h. mit Faktoren, die den Wundheilungsprozess behindern, wobei man diese zusätzlichen Substanzen kovalent in das Hydrogel einpolymerisiert. Die Erfindung beschreibt die neuartige Herstellung von proteinhaltigen Hydrogelen, mit denen es möglich ist, reaktive ROS in der Wundflüssigkeit von chronischen Wunden unschädlich zu machen. Sie beschreibt das Herstellen von collagenasehaltigen und trypsinhaltigen Hydrogelen zur Behandlung von nekrotischen Wundbelägen, sowie das Herstellen von lysozymhaltigen Hydrogelen, die zur Bekämpfung von mit Bakterien infizierten Wunden eingesetzt werden können. Ferner wird die Machbarkeit beschrieben, in die Hydrogele biologisch aktive Substanzen wie zum Beispiel Antibioüka, Wachstumsfaktoren und andere Wirkstoffe einzuarbeiten.

Die vorliegende Erfindung bedient sich der Reaktion von insbesondere $\alpha,\omega$-Diisocyanato-Polyethylenglykolen mit Protein unter Bildung eines proteinhaltigen Hydrogels im wäßrigen Milieu. Dabei ist die Vernetzungsreaktion des Isocyanats mit den Aminogruppen des Proteins eine durch die höhere Nucleophilie bevorzugte Reaktion, im Vergleich zur Hydrolyse des Isocyanats zum Amin unter Bildung von Kohlendioxid. Die Reaktion stellt eine Möglichkeit zur dreidimensionalen Vernetzung von Polyethylenglykol und Proteinen dar ohne das Auftreten von Spaltprodukten, die aus der aktivierten Gruppe entstehen. Ferner können stabile Hydrogele mit oligomeren Proteinen wie zum Beispiel SOD und Katalase gebildet werden.

Polyethylenglykole (PEGs) zählen zur Klasse der Polyether gehörenden Polyalkylenglykole der allgemeinen Formel (Römpp Lexikon Chemie - Version 1.3, Stuttgart/New York: Georg Thieme Verlag 1997):

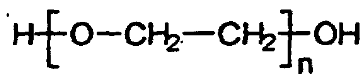

Polyethylenglykole (PEGs) werden technisch hergestellt durch basisch katalysierte Polyaddition von Ethylenoxid (Oxiran) in meist geringe Mengen Wasser enthaltenden Systemen mit Ethylenglykol als Startmolekül. Sie haben Molmassen im Bereich von ca. 200-5 000 000 g/mol, entsprechend Polymerisationsgraden n von ca. 5 bis >100 000. Im weiteren Sinne werden auch Produkte mit n=2-4 (Di-, Tri- u. Tetraethylenglykol) zu den PEGs gerechnet; sie sind mol.-einheitlich herstellbar, während die PEGs mit höheren Molmassen polymol. sind, d. h. aus Kollektiven von Makromolekülen mit unterschiedlichen Molmassen bestehen.

Flüssige Produkte mit Molmassen <ca. 25 000 g/mol werden als eigentliche Polyethylenglykole, die höhermolekularen festen (Schmelzpunkt ca. 65 °C) als Polyethylenoxide bezeichnet. Polyethylenoxide besitzen eine äußerst niedrige Konzentration an reaktiven Hydroxy-Endgruppen und zeigen nur noch schwache Glykol-Eigenschaften.

Als PEG werden auch verzweigte Polyaddukte von Ethylenglykol an mehrwertige Alkohole bezeichnet.

PEG sind flüssige bzw. wachsartige bis feste Produkte, die sich in Wasser bis ca. 100 °C und in vielen organischen Lösungsmitteln gut lösen. Wäßrige Lösungen haben auffallende rheologische Eigenschaften, so zeigen verschiedene Lösungen zum Teil starke Viskoelastizität. In fließendem Wasser bewirken schon minimale PEG-Mengen den sogenannten Toms-Effekt (Herabsetzung des Reibungswiderstands). PEG sind sehr hydrolysestabile, bei höheren Temperaturen aber oxididationsempfindliche Produkte. Ihre chemische Reaktivität wird durch die terminalen Hydroxy-Gruppen bestimmt, die leicht verestert (zu Polyethylenglykolester) oder verethert (zu Polyalkylenglykolether) oder mit Isocyanaten zu Urethanen umgesetzt werden können.

PEG werden als toxikologisch unbedenklich eingestuft. Ihre biologische Abbaubarkeit ist stark Molmassenabhängig; Produkte mit niedrigen Molmassen, z. B. 4000 g/mol, wer den bis zu 80% abgebaut.

PEGs werden u.a. verwendet als Lösungsvermittler, Bindemittel, Konsistenzgeber, Emulgatoren, Dispergatoren, Schutzkolloide, Weichmacher oder Trennmittel für sehr unterschiedliche Einsatzgebiete; als Bindemittel für keramische Massen, Schlichtemittel, Flockungsmittel, Klebstoff-Komponenten, zur Verminderung des Fließwiderstands wäßriger Flüssigkeiten (drag-reduction), als Zwischenprodukte für Polymer-Synthesen, zum Beispiel im großen Umfang für die Herstellung von Polyurethanen; hochmolekulare PEGs als Stärkeersatz sowie zur Herstellung von Filmen und Folien.

Vorteilhafterweise hat das eingesetzte PEG ein Molgewicht von 8.000 bis 18.000 g/mol, insbesondere 10.000 bis 15.000 g/mol.

Erfindungsgemäß können auch modifizierte PEGs eingesetzt werden, die die folgende Strukturschemata aufweisen können:

A—B—A

(1)

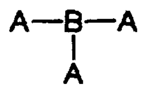

(2)

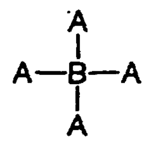

(3)

wobei B einen hydrophilen Bereich des jeweiligen Vernetzermoleküls symbolisiert und A eine Isocyanatgruppe darstellt, welche auch innerhalb eines Moleküls unterschiedlicher chemischer Natur sein mögen.

Ebenfalls in den Rahmen der hiermit vorgelegten Erfindung fallen Struktur-schemata wie folgt:

A—B—Z—B—A

(9)

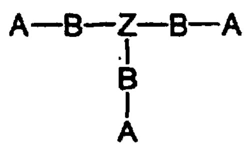

(10)

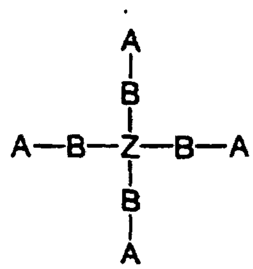

(11)

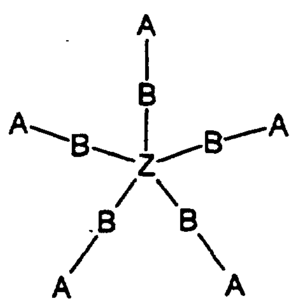

(12)

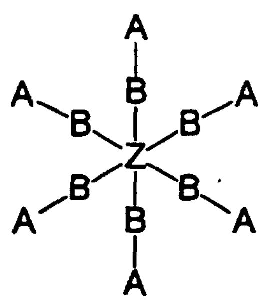

(13)

wobei Z dabei eine Zentraleinheit darstellt, welche hydrophil oder hydrophob sein kann und in der Regel aus einem oligo- oder polyfunktionellen Molekülrest besteht.

Selbstverständlich fallen auch Verknüpfersubstanzen mit höherem Verzweigungsgrad in den Rahmen der vorliegenden Erfindung.

Beispielsweise kann Z in Schema (10) aus einem Glycerylrest bestehen, dessen drei OH-Funktionen in die Bereiche B übergehen, welche ihrerseits beispielsweise Polyoxyethylenketten gleicher oder ungleicher Länge darstellen können, und deren terminale OH-Gruppe mit einer längerkettigen Fettsäure verestert ist. Auch Teilsubstitution an Glycerin ist denkbar, wodurch Strukturen entstehen können, welche Schema (9) entsprechen.

Für das Strukturschema (1) können beispielsweise folgende spezielleren Struktur schemata befolgt werden:

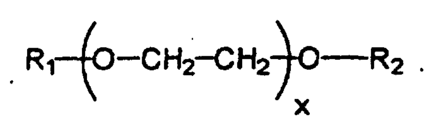

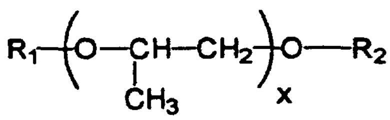

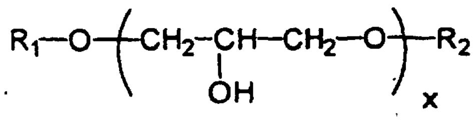

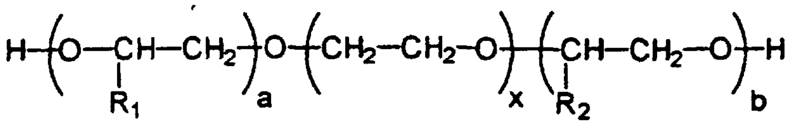

wobei $R_1$, $R_2$, $R_3$, $R_4$, $R_5$ und $R_6$ unabhängig voneinander Isocyanatgruppen oder verzweigte oder unverzweigte, gesättigte oder ungesättigte, cyclische oder kettenförmige aliphatische, aromatische oder heteroaromatische Reste darstellen können, beispielsweise verzweigte oder unverzweigte oder cyclische Alkyl- oder Alkanoylreste, mit Alkyl- oder Arylsubstituenten substituierte oder unsubstituierte Aryl- oder Aroylreste oder auch alkylierte oder arylierte Organylsilylreste bedeuten, wobei aber mindestens einer dieser Reste eine Isocyanatgruppe darstellt. x, y und z bedeuten dabei unabhängig voneinander Zahlen, die es dem Gesamtmolekül erlauben, in Wasser löslich oder zumindest dispergierbar zu sein, typischerweise gewählt aus dem Bereich größer als 10, vorteilhaft aus dem Bereich 20 bis $10^7$. a und b sind Zahlen, die in Abhängigkeit von x gewählt werden, dergestalt, daß die Verknüpfersubstanz eine wenigstens ausreichende Wasserlöslichkeit bzw. Wasserdispergierbarkeit aufweist. Im Einzelfalle, beispielsweise wenn der Verdicker aus der Gruppe der derivatisierten Polysaccharide gewählt wird, kann x gegebenenfalls wesentlich höhere Werte als z.B. 300, sogar mehrere Millionen, annehmen. Dies ist dem Fachmanne an sich bekannt und bedarf keiner weiteren Erläuterung.

Für das Strukturschema (2) können beispielsweise folgende spezielleren Strukturschemata befolgt werden:

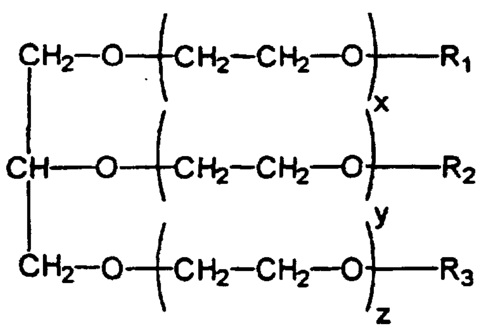

wobei $R_1$, $R_2$ und $R_3$ unabhängig voneinander Isocyanatgruppen oder verzweigte oder unverzweigte, gesättigte oder ungesättigte, cyclische oder kettenförmige aliphatische, aromatische oder heteroaromatische Reste darstellen können, beispielsweise verzweigte oder unverzweigte oder cyclische Alkyl- oder Alkanoylreste, mit Alkyl- oder Arylsubstituenten substituierte oder unsubstituierte Aryl- oder Aroylreste oder auch alkylierte oder arylierte Organylsilylreste bedeuten, wobei aber mindestens einer dieser Reste eine Isocyanatgruppe darstellt. x, y und z bedeuten dabei unabhängig voneinander Zahlen, die es dem Gesamtmolekül erlauben, in Wasser löslich oder zumindest dispergierbar zu sein, typischerweise gewählt aus dem Bereich größer als 10, vorteilhaft aus dem Bereich 20 bis $10^7$.

Auch Teilsubstitution ist dabei denkbar, wobei einer oder mehrere der Indices x, y, oder z den Wert Null annehmen können und einer oder mehrere der Reste $R_1$, $R_2$ oder $R_3$ Wasserstoffatome darstellen können.

Für das Strukturschema (3) können beispielsweise folgende spezielleren Strukturschemata befolgt werden:

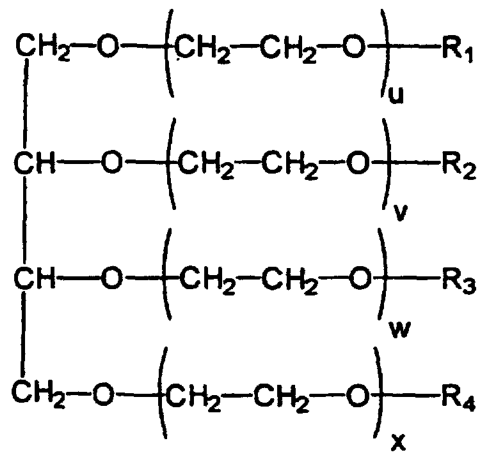

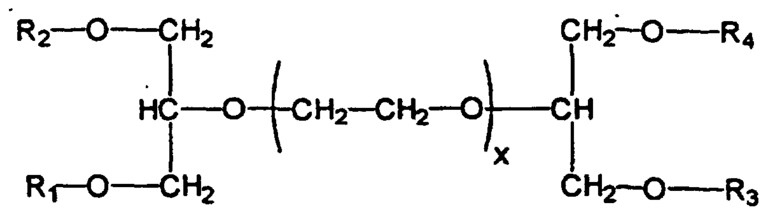

wobei $R_1$, $R_2$, $R_3$ und $R_4$ unabhängig voneinander Isocyanatgruppen oder verzweigte oder unverzweigte, gesättigte oder ungesättigte, cyclische oder kettenförmige aliphatische, aromatische oder heteroaromatische Reste darstellen können, beispielsweise verzweigte oder unverzweigte oder cyclische Alkyl- oder Alkanoylreste, mit Alkyl- oder Arylsubstituenten substituierte oder unsubstituierte Aryloder Aroylreste oder auch alkylierte oder arylierte Organylsilylreste bedeuten, wobei aber mindestens einer dieser Reste eine Isocyanatgruppe darstellt. x, y und z bedeuten dabei unabhängig voneinander Zahlen, die es dem Gesamtmolekül erlauben, in Wasser löslich oder zumindest dispergierbar zu sein, typischerweise gewählt aus dem Bereich größer als 10, vorteilhaft aus dem Bereich 20 bis $10^7$.

Auch hier gilt selbstverständlich, daß Teilsubstitution denkbar ist, wobei einer oder mehrere der Indices u, v, w, x den Wert Null annehmen können und einer oder mehrere der Reste $R_1$, $R_2$ ,$R_3$ oder $R_4$ Wasserstoffatome darstellen können. Dabei gehen die Substanzen natürlich in andere Strukturschemata über.

Für das Strukturschema (9) können beispielsweise folgende spezielleren Strukturschemata befolgt werden:

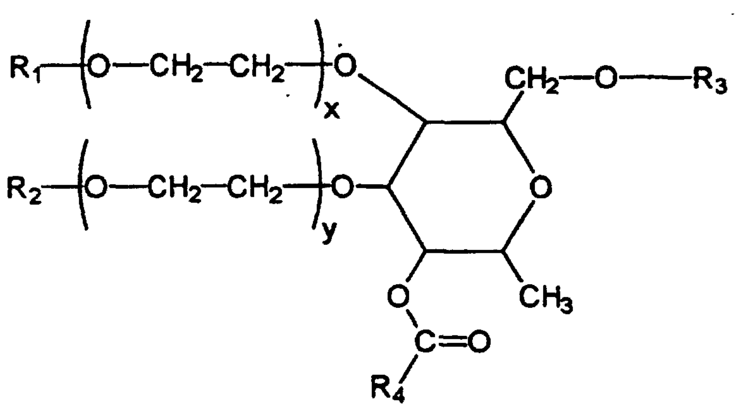

wobei $R_1$, $R_2$, $R_3$ und $R_4$ unabhängig voneinander Isocyanatgruppen oder verzweigte oder unverzweigte, gesättigte oder ungesättigte, cyclische oder kettenförmige aliphatische, aromatische oder heteroaromatische Reste darstellen können, beispielsweise verzweigte oder unverzweigte oder cyclische Alkyl- oder Alkanoylreste, mit Alkyl- oder Arylsubstituenten substituierte oder unsubstituierte Aryloder Aroylreste oder auch alkylierte oder arylierte Organylsilylreste bedeuten, wobei aber mindestens einer dieser Reste eine Isocyanatgruppe darstellt. x, y und z bedeuten dabei unabhängig voneinander Zahlen, die es dem Gesamtmolekül erlauben, in Wasser löslich oder zumindest dispergierbar zu

sein, typischerweise gewählt aus dem Bereich größer als 10, vorteilhaft aus dem Bereich 20 bis $10^7$.

Für das Strukturschema (10) können beispielsweise folgende spezielleren Strukturschemata befolgt werden:

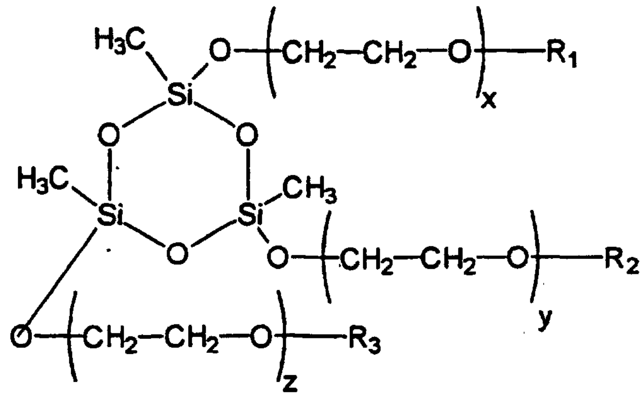

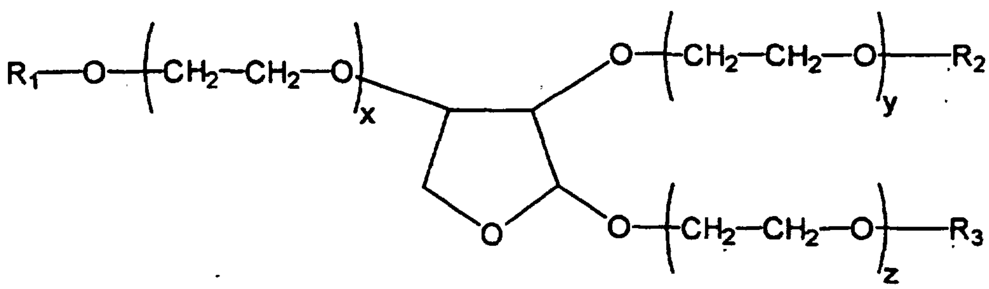

wobei $R_1$, $R_2$, und $R_3$ unabhängig voneinander Isocyanatgruppen oder verzweigte oder unverzweigte, gesättigte oder ungesättigte, cyclische oder kettenförmige aliphatische, aromatische oder heteroaromatische Reste darstellen können, beispielsweise verzweigte oder unverzweigte oder cyclische Alkyl- oder Alkanoylreste, mit Alkyl- oder Arylsubstituenten substituierte oder unsubstituierte Aryl- oder Aroylreste oder auch alkylierte oder arylierte Organylsilylreste bedeuten, wobei aber mindestens einer dieser Reste eine Isocyanatgruppe darstellt. x, y und z bedeuten dabei unabhängig voneinander Zahlen, die es dem Gesamtmolekül erlauben, in Wasser löslich oder zumindest dispergierbar zu sein, typischerweise gewählt aus dem Bereich größer als 10, vorteilhaft aus dem Bereich 20 bis $10^7$.

Für das Strukturschema (11) kann beispielsweise folgendes speziellere Strukturschema befolgt werden:

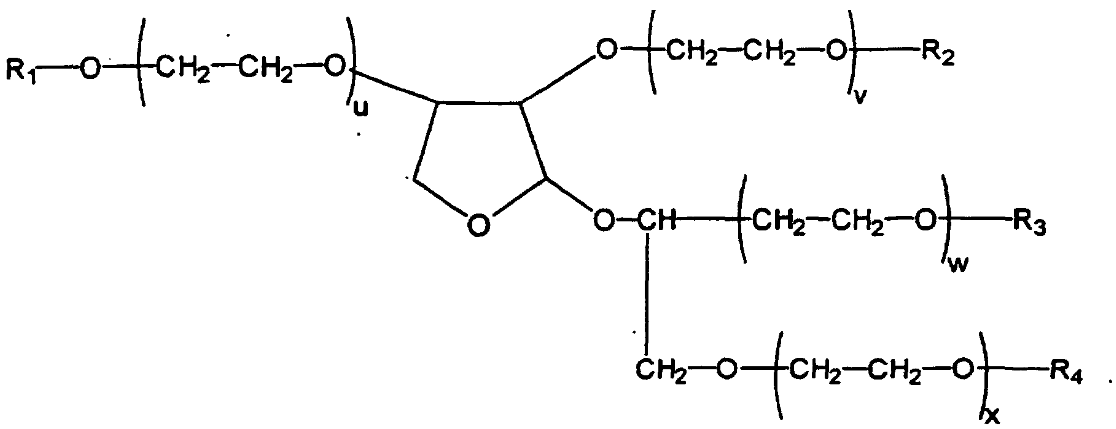

wobei $R_1$, $R_2$, $R_3$ und $R_4$ unabhängig voneinander Isocyanatgruppen oder verzweigte oder unverzweigte, gesättigte oder ungesättigte, cyclische oder kettenförmige aliphatische, aromatische oder heteroaromatische Reste darstellen können, beispielsweise verzweigte oder unverzweigte oder cyclische Alkyl- oder Alkanoylreste, mit Alkyl- oder Arylsubstituenten substituierte oder unsubstituierte Aryloder Aroylreste oder auch alkylierte oder arylierte Organylsilylreste

bedeuten, wobei aber mindestens einer dieser Reste eine Isocyanatgruppe darstellt. x, y und z bedeuten dabei unabhängig voneinander Zahlen, die es dem Gesamtmolekül erlauben, in Wasser löslich oder zumindest dispergierbar zu sein, typischerweise gewählt aus dem Bereich größer als 10, vorteilhaft aus dem Bereich 20 bis $10^7$.

Für das Strukturschema (12) kann beispielsweise folgendes speziellere Strukturschema befolgt werden:

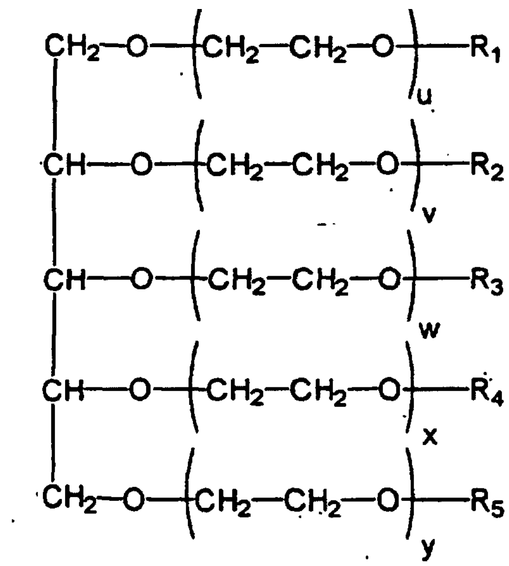

wobei $R_1$, $R_2$, $R_3$, $R_4$ und $R_5$ unabhängig voneinander Isocyanatgruppen oder verzweigte oder unverzweigte, gesättigte oder ungesättigte, cyclische oder kettenförmige aliphatische, aromatische oder heteroaromatische Reste darstellen können, beispielsweise verzweigte oder unverzweigte oder cyclische Alkyl- oder Alkanoylreste, mit Alkyl- oder Arylsubstituenten substituierte oder unsubstituierte Aryloder Aroylreste oder auch alkylierte oder arylierte Organylsilylreste bedeuten, wobei aber mindestens einer dieser Reste eine Isocyanatgruppe darstellt. x, y und z bedeuten dabei unabhängig voneinander Zahlen, die es dem Gesamtmolekül erlauben, in Wasser löslich oder zumindest dispergierbar zu sein, typischerweise gewählt aus dem Bereich größer als 10, vorteilhaft aus dem Bereich 20 bis $10^7$.

Für das Strukturschema (13) kann beispielsweise folgendes speziellere Strukturschema befolgt werden:

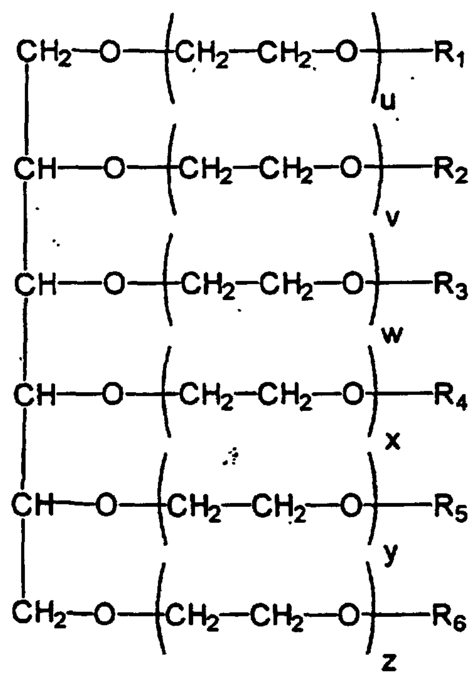

wobei $R_1$, $R_2$, $R_3$, $R_4$, $R_5$ und $R_6$ unabhängig voneinander Isocyanetgruppen oder verzweigte oder unverzweigte, gesättigte oder ungesättigte, cyclische oder kettenförmige aliphatische, aromatische oder heteroaromatische Reste darstellen können, beispielsweise verzweigte oder unverzweigte oder cyclische Alkyl- oder Alkanoylreste, mit Alkyl- oder Arylsubstituenten substituierte oder unsubstituierte Aryl- oder Aroylreste oder auch alkylierte oder arylierte Organylsilylreste bedeuten, wobei aber mindestens einer dieser Reste eine Isocyanatgruppe darstellt. x, y und z bedeuten dabei unabhängig voneinander Zahlen, die es dem Gesamtmolekül erlauben, in Wasser löslich oder zumindest dispergierbar zu sein, typischerweise gewählt aus dem Bereich größer als 10, vorteilhaft aus dem Bereich 20 bis $10^7$.

Es ist auch gegebenenfalls von Vorteil, die vorab beschriebenen Strukturschemata so abzuwandeln, daß am Ende des Verdickermoleküls erneut Verzweigung auftritt, . etwa dergestalt, wie es in der Gruppe der sogenannten Dendrimere verwirklicht wird.

Die Wahl des zur Herstellung des Hydrogels erforderlichen Proteins ist abhängig von den gewünschten Eigenschaften des Hydrogels. Prinzipiell eignen sich alle Proteine und Enzyme sowie Teile von Proteinen und Enzymen oder rekombinant hergestellte Proteine und Enzyme wie beispielsweise:

- Antikörper
- Radikale detoxifizierende Enzyme wie z.B. Superoxiddismutase, Katalase, Glutathion-Peroxidase, Myeloperoxidase und/oder Kombinationen daraus sowie SOD/Katalase-Enzym-Mimics
- proteolytisch wirkende Enzyme wie Collagenase, Trypsin, Elastase und/oder Kombinationen daraus
- Proteaseinhibitoren aus der Klasse der Tissue Inhibitors of Matrixmetalloproteinases (TIMPs) und/oder andere proteinogene Proteaseinhibitoren wie Aprotinin, Sojabohnen-Trypsininhibitor und Alpha-2-Makroglobulin
- antimikrobiell wirksame Enzyme wie zum Beispiel Lysozym und Hydrolase
- phosphorylierend wirksame Enzyme wie Phosphatasen und Kinasen
- Wachstumsfaktoren wie z.B. PDGF
- beliebige Mischungen der Proteine, wobei sich die Zusammensetzung der Mischung ebenfalls an den gewünschten funktionalen Eigenschaften des Hydrogels orientiert

Als Enzyminhibitoren werden besonders natürliche proteinogene Protease-Inhibitoren aus der Klasse der TIMPs gewählt sowie Aprotinin, Alpha-2-Antiplasmin, Alpha-2-Makroglobulin, Alpha-1-Antichymotrypsin, Sojabohnen-Trypsininhibitor und Alpha-1-Protease-Inhibitor.

Im Falle eines proteinhaltigen Hydrogels, das aus SOD, Katalase oder aus einer Mischung der beiden Enzyme besteht, ist ein selektives Entfernen von ROS aus der Wundflüssigkeit möglich. ROS werden in der entzündlichen Phase der Wundheilung durch Neutrophile und Monozyten nach einem zellulären Reiz ausgeschüttet. Das Enzym SOD katalysiert die Dismutationsreaktion von reaktivem Superoxid in die weniger toxische Zwischenstufen Wasserstoffperoxid und Sauerstoff (Gleichung 1). Es spielt die Hauptrolle in der zellulären Verteidigung gegen die sauerstoffvermittelte Toxizität von ROS und bei der Regulierung der intrazellulären Sauerstoffkonzentration (Fridovich, 1995, Annu. Rev. Biochem., 64, 97). Das durch die Reaktion der SOD gebildete Wasserstoffperoxid wird anschließend durch das in aeroben Organismen ubiquitäre Redoxenzym Katalase in einem mehrstufigen katalytischen Zyklus in die nichttoxischen Moleküle Wasser und Sauerstoff (Gleichung 2) umgewandelt (Gouet et al., 1996, Nature Structural Biology, 3, 951). Außer Katalase sind auch die Enzyme Gluthathion-Peroxidase und Myeloperoxidase befähigt, Wasserstoffperoxid abzubauen.

$$2\ O_2^- + 2\ H^+ \rightarrow H_2O_2 + O_2 \quad (1)$$

$$H_2O_2 \rightarrow H_2O + \tfrac{1}{2}\ O_2 \quad (2)$$

Collagenasehaltige Salben werden seit Jahren zur Entfernung von nekrotischen Wundbelägen eingesetzt (Nano et al., 1996, in: Proteolysis in Wound Repair, Abatangelo, Donati und Vanscheidt (Eds.), Springer Verlag, Berlin, Seite 61) und J. Hansbrough & W. Hansbrough 1996, in: Proteolysis in Wound Repair, Abatangelo, Donati und Vanscheidt (Eds.), Springer Verlag, Berlin, Seite 97. Das Enzym Collagenase und andere Proteasen spalten dabei Collagenmoleküle in abgestorbenem Gewebe und machen somit das Einwandern von Fibroblasten möglich und somit das Abheilen der Wunde (Glyantsev et al., 1997, J. Wound Care, 6, 13). Ein aus Collagenase bestehendes Hydrogel trägt in vergleichbarer Art und Weise zur Auflösung des nekrotischen Gewebes bei.

Die aktiven Enzyme werden beim Entfernen des Hydrogels ebenfalls mit entfernt, ohne daß sie in der Wunde bzw. in der Wundflüssigkeit verbleiben. Erfindungsgemäß schließt diese Wechselwirkung eine Umwandlung der ROS als Störfaktoren in die Wundheilung nicht mehr behindernde Substanzen ein. Die zur Wechselwirkung verwendeten, kovalent in das Hydrogel eingebundenen Enzyme werden somit nur vorübergehend in die Wunde eingebracht und werden, nachdem sie ihre bestimmungsgemäße Aufgabe verrichtet haben (d. h., die oben genannten Wechselwirkungen eingegangen sind), wieder aus dem Wundbereich entfernt. Durch die selektive Beseitigung bzw. Eliminierung der toxischen ROS, das proteolytische Auflösen von totem Gewebe und das Abtöten von Mikroorganismen wird der Heilungsprozeß chronischer, d. h. schwer oder nicht heilender, Wunden verbessert bzw.- eingeleitet.

In der chronischen Wunde findet man eine deutlich erhöhte Proteaseaktivität (Weckroth et al., 1996, J. Invest. Dermatol., 106, 1119; Grinell & Zhu, 1996, J. Invest. Dermatol. 106, 335) im Vergleich zur akuten Wunde. Dem steht eine deutlich verringerte Menge an Proteaseinhibitoren gegenüber (Grinell & Zhu, 1996, J. Invest. Dermatol. 106, 335). Durch dieses Ungleichgewicht kommt es zu einem proteolytischen Abbau von proliferationsfördernden Substanzen wie zum Beispiel Wachstumsfaktoren (Wlaschek et al., 1997, Brit. J. Dermatol., 137, 646). Enzyme wie zum Beispiel SOD und Katalase die durch ihre schützende Wirkung wundheilungsfördernd wirken, würden dadurch proteolytisch abgebaut und somit unwirksam. Ein proteinhaltiges Hydrogel in das solche Enzyme kovalent eingearbeitet sind, wirkt als Schutzschild und kann den Angriff von Proteasen verhindern, wie mit Polyethylenglykol modifizierten gelösten Enzymen gezeigt wurde (Beckman et al., 1988, J. Biol. Chem., 14, 6884; Inada et al., 1995, Tibtech, 13, 86).

Im Rahmen der vorliegenden Erfindung ist es somit nicht nur möglich, ein feuchtes Wundmilieu zu generieren, um den Heilungsprozeß chronischer Wunden zu verbessem, sondern der Heilungsprozeß kann auch erfindungsgemäß dadurch weiter beschleunigt werden, zum Beispiel durch die oben genannten Urnwandlungsprozesse.

Anschließend werden Verfahren zur Herstellung des erfindungsgemäßen Hydrogeles näher erläutert.

In übersichtlicher Form stellt sich ein vorteilhaftes Verfahren zur Herstellung der erfindungsgemäßen Hydrogele wie folgt dar:

a) Wasserfreie PEGs werden in Lösungsmittel mit Diisocyanat gegebenenfalls unter Zusatz eines Katalysators umgesetzt,

b) das resultierende Produkt aus aktiviertem PEGs wird vom Lösungsmittel durch Abfiltem, Waschen oder Trocknung befreit,

c) die aktivierten PEGs werden in wäßriger Lösung mit Proteinen umgesetzt, wobei sich die Proteine in einem Puffer befinden, der bevorzugt so gewählt ist, daß die Proteine ihre biologische Aktivität erhalten,

d) gegebenenfalls werden Reinigungsschritte und Waschungen vorgenommen.

Vorzugsweise wird das Hydrogel anschließend dehydratisiert.

Das erfindungsgemäße Hydrogel ist besonders vorteilhaft als Wundauflage insbesondere für tiefe und flächige chronische Wunden sowie Brandwunden geeignet.

Des weiteren zeigt das Hydrogel vorteilhafte Eigenschaften als Auflage auf gegebenenfalls luft- und wasserdampfdurchlässigen Trägermaterialien wie Binden, Kompressen, Pflastern, Folien, Filmen und dergleichen.

Im folgenden werden die einzelnen Verfahrensschritte intensiver beschrieben.

Herstellung von aktivierten $\alpha,\omega$-Diisocyanato-Polyethylenglykolen

Die Experimente zur Herstellung eines aktivierten Polyethylenglykols werden wie folgt beschrieben durchgeführt.
Polyethylenglykole verschiedener Molmassen (6000 - 35000 g/mol) werden durch kovalente Verknüpfung von aliphatischen Diisocyanaten, unter Ausbildung von Urethanbindungen in $\alpha,\omega$-Diisocyanato-Polyethylenglykolen überführt. Um eine Hydrolyse der eingesetzten Isocyanatgruppen zu vermeiden, wird das PEG vor der eigentlichen Verwendung dehydriert. Dazu werden 1.0 mmol PEG in 60 ml Benzol gelöst und in flüssigem Stickstoff eingefroren. Das dabei gebildete Benzol-Wasser Azeotrop wird für 7 h im Hochvakuum abgetrennt. 1.0 mmol des so dehydratisierten PEG werden unter Aufrechterhaltung einer Argonatmosphäre in 50 ml *abs*. Dichlormethan gelöst. Zu dieser Lösung werden beispielsweise 10 mmol Hexamethylendiisocyanat und 1 ml *abs*. Pyridin gegeben und für 12 h bei RT gerührt. Die Reaktionslösung wurde durch zweimaliges Umfällen in 800 ml *abs*. Petrolether 35/60 aufgearbeitet. Das kristalline

Produkt wurde über einen Büchnertrichter abgefiltert und mit *abs*. Petrolether 35/60 (2 x 100 ml) gewaschen. Nach 8 h Trocknung im Hochvakuum wird das aktivierte PEG in einer Argonatmosphäre bei - 20 °C gelagert.

Für die Aktivierung der PEG können erfindungsgemäß neben 1,6-Hexamethylendiisocyanat auch andere aliphatische oder aromatische Diisocyanate eingesetzt werden, beispielsweise 1,12-Dodecandiisocyanat, Isophorondiisocyanat, Methylcyclohexan-2,4- und/oder -2,6-diisocyanat, Dicyclohexylmethan-2,4'- und/oder -4,4'-diisocyanat ferner Cyclobutan-1,3-diisocyanat, Cyclohexan-1,3- und 1,4-diisocyanat, 2,4-und/oder 2,6 Hexahydrotoluylendiisocyanat, Hexahydro-1,3- und/oder -1,4-phenylendiisocyanat sowie 1,3- und 1,4-Phenylendiisocyanat, 2,4- und 2,6-Toylendiisocyanat, Diphenylmethan-2,4'- und/oder -4,4'-diisocyanat und Naphthylen-1,5-diisocyanat.

Herstellung von proteinhaltigen Hydrogelen

Überraschenderweise wurde gefunden, daß die neuheitliche Reaktion der $\alpha,\omega$-Diisocyanato-Polyethylenglykole mit Proteinen unter Bildung proteinhaltiger Hydrogele im Vergleich zu der 4-Nitrophenylchloroformiat-Aktivierung mit erheblich kürzeren Gelbildungszeiten verbunden ist.

Figur 1 ist die graphische Darstellung der Abhängigkeit der Gelbildungszeit in Abhängigkeit vom Molekulargewicht der eingesetzten $\alpha,\omega$-Diisocyanato-Polyethylenglykole und dem pH-Wert der Reaktionslösung. Dabei ist auf der x-Achse das Molekulargewicht der eingesetzten PEG in Gramm/Mol und auf der y-Achse die Gelbildungszeit in Sekunden dargestellt. Desweiteren sind drei 0.1 M Boratpufferlösungen unterschiedlicher pH-Werte dargestellt (O = pH 9.5, □ = pH 9.0, Δ = pH 8.5).

Figur 2 ist die graphische Darstellung der Abhängigkeit der maximal aufnehmbaren Wassermenge von der Ionenstärke der Inkubationslösung. Der prozentuale Wasseranteil am Gesamtgewicht der Hydrogele ist auf der x-Achse dargestellt, während auf der y-Achse die Ionenstärke der Inkubationslösung in Form der Boratkonzentration , aufsteigend von 0 - 0.2 Mol pro Liter, aufgeführt ist. Der dargestellte Versuch erfolgte mit Hydrogelen der Zusammensetzung 100 mg $PEG_{15000}$ und 35 mg BSA.

Wie oben beschrieben, war es bisher nicht möglich, andere als albuminartige Proteine isoliert, in dreidimensionaler Form, innerhalb einer Hydrogelmatrix zu immobilisieren. Erfolgte eine beobachtbare Gelbildung mit anderen Proteinen bzw. Enzymen., lösten sich diese innerhalb weniger Minuten wieder auf (siehe US 5,733,563) und lediglich eine Immobilisierung als ternäres System, bestehend aus PEG/BSA/Enzym, konnte erzielt werden (Fortier et al.1996. Enz. Microbiol.Technol 18, 482).

Überraschenderweise stellte sich weiterhin heraus, daß die beschriebene Form der PEG-Aktivierung über Diisocyanate, eine Immobilisierung anderer Proteine und Enzyme, als albuminartige, zuläßt, so Superoxiddismutase (SOD) aus Rind und Hefe. Hierzu werden 44 mg Superoxiddismutase in 2 ml 0.1 m Boratpuffer gelöst und mit 150 mg PEG verschiedener Molekulargewichte versetzt. Nach erzielter Vernetzung werden die SOD-Hydrogele analog zu den BSA-Hydrogelen durch Inkubation in physiologischem Boratpuffer gewaschen und die Waschlösung auf unvemetztes PEG und Enzym hin untersucht. Hierbei wird festgestellt, daß unter bestimmten Voraussetzungen eine Gelbildung erfolgt, so daß sich ein stabiles SOD-Hydrogel ergibt. Als ein wesentlicher Faktor erweist sich dabei das Molekulargewicht und damit die Kettenlänge, der eingesetzten Polyethylenglykole. Oberhalb eines Molekulargewichtes von $PEG_{20000}$ ist eine Gelbildung nur mehr schwerlich möglich.

Um eine Abschätzung über die Beibehaltung der Enzymaktivität zu erhalten, werden Proben der hergestellten SOD-Hydrogele mithilfe des NBT-Nachweises (Auclair and Voisin, 1985, in: CRC Handbook of Methods for Oxygen Radical Research, CRC Press Inc., Boca Raton) hinsichtlich ihrer Aktivität untersucht. Da sich dieser Test nur zur quantitativen Bestimmung gelöster SOD eignet, lassen sich damit nur qualitative Aussagen über das Vorhandensein von Aktivität immobilisierter SOD treffen. Die erzielten Ergebnisse lassen jedoch darauf schließen, daß die Immobilisierung innerhalb der Gelmatrix keinen signifikanten Einfluß auf die Aktivität des Enzyms zu nehmen scheint. Auch nach mehrwöchiger Lagerung bei unterschiedlichen Temperaturen (4 °C, 25 °C, 37 °C), anschließender Dehydratisierung im Hochvakuum und erneutem Quellen in Inkubationslösung, kann eine unverändert vorhandene Enzymaktivität beobachtet werden.

Ausgehend von diesen Ergebnissen wurden die Versuche auf andere Enzyme ausgeweitet. Dabei wurde die Vernetzungsmöglichkeit folgender Enzyme getestet:

- Katalase aus Rind
- Lysozym aus Hühnereiweiß und
- Collagenase aus *achromobacter iophagus*.

Mit allen genannten Enzymen kann ein stabiles Hydrogel erzielt werden, wobei der limitierende Faktor die Kettenlänge

des eingesetzten PEG-Molekül ist.

**Tabelle 1:**

**Vernetzungsmöglichkeiten von verschiedenen Proteinen und Enzymen mit α,ω-Diisocyanato-PEG.**

**Alle Versuche erfolgten in 2 ml 0.1m Boratpuffer (pH 9.0) mit 150 bis 300 mg PEG und 35 bis 100 mg Protein/Enzym.**

| | $PEG_{15000}$ | $PEG_{10000}$ | $PEG_{6000}$ |
|---|---|---|---|
| BSA | x | x | x |
| SOD | x | x | x |
| Katalase | x | x | x |
| Collagenase | x | x | |
| Lysozym | x | x | x |
| Phosphatase | | x | x |
| Trypsin | | x | x |
| SOD/Katalase | x | x | x |

Methode zur Waschung der proteinhaltigen Hydrogele

Nach vollendeter Vernetzung werden die Hydrogele bei RT in 15 ml physiologischem Boratpuffer (0.1 m $H_3BO_3$, 0.1 m $MgCl_2$, 0.1 m $CaCl_2$, 0.15 m NaCl; pH 7.0) inkubiert. Nach jeweils 12 Stunden wird die Inkubationslösung gewechselt und der erhaltende Überstand auf ausgewaschenes Protein und PEG hin untersucht. Die Evaluation der resultierenden Mengen an Protein erfolgt mithilfe des BCA- und Micro BCA-Nachweises (Rhoderick, Jarvis and Hyland, 1989, Anal. Biochem., 180, 136) und die Bestimmung der eluierten Mengen an PEG mithilfe des Jod-Nachweises (Sims and Snape. 1980, Anal. Biochem., 107, 60).
Das Ende der Waschung ist dann erreicht, wenn weder Protein noch PEG in der Waschlösung detektierbar sind (zwischen 48 - 72 h Inkubation). Anschließend werden die Hydrogele entweder in dehydratisierter (siehe Methode zur Dehydratisierung proteinhaltiger Hydrogele) oder in gequollener Form bei RT gelagert

Methode zur Dehydratisierung der proteinhaltigen Hydrogele

Nach Abschluß des Waschvorganges werden die Gele im Hochvakuum bis zur Gewichtskonstanz dehydratisiert und erneut in Pufferlösungen inkubiert, um so die Abhängigkeit der Wasseraufnahme von verschiedenen Faktoren wie pH-Wert und Ionenstärke der Hydrogele zu untersuchen. Das Quellen der Gele wird, bis zum Erreichen der maximalen Wasseraufnahmekapazität, durch regelmäßige Gewichtsbestimmung verfolgt, wobei die Inkubationslösung alle 12 h gewechselt wird. Der Quellfaktor (SF) [Gleichung 3] und der maximale Wassergehalt (EWC) [Gleichung 4] werden durch zwei mathematische Beziehungen beschrieben, die zur Bestimmung des Quellverhaltens von Polyvinylalkohol-Hydrogelen benutzt werden (Urushisaki et al., 1990, Int. J. Pharm., 58, 135):

$$\text{Quellfaktor (SF)} = (W_s - W_d)/W_d \qquad (3)$$

Hierbei entspricht $W_s$ dem maximal erreichbaren Quellgewicht und $W_d$ dem Trockengewicht der Hydrogele. Dieses Trockengewicht wird nach Abschluß der Untersuchungen durch Trocknung der Gele bei 70 °C, über einen

Zeitraum von 24 h bestimmt.
Aus diesen gewonnenen Daten läßt sich auch der prozentuale Anteil des Wassers am Gesamtgewicht der Hydrogele bestimmen:

$$EWC = (\text{Gewicht Wasser}/\text{Gewicht gequollenes Hydrogel}) \times 100 \qquad (4)$$

Versuche hinsichtlich des Quellverhaltens bei unterschiedlichen pH-Werte und Ionenstärken der Inkubationslösungen haben gezeigt, daß ein direkter Zusammenhang zwischen diesen Parametern und dem Quellvermögen der Hydrogele besteht Bewirkt zum Beispiel die Erhöhung der Ionenstärke der Inkubationslösung eine Abnahme der Wasseraufnahmekapazität, so läßt sich bei der Variation des pH-Wertes der Inkubationslösung der gegenläufige Effekt beobachten. Hier führt die Erhöhung des pH-Wertes zu einer Zunahme der Wasseraufnahmekapazität.
Der Einfluß des Molekulargewichtes der eingesetzten Polyethylenglykole auf das Quellverhalten der resultierenden Hydrogele ist in Tabelle 1 detailliert aufgeführt

Tabelle 2:

Evaluierung der Quellparameter (Quellfaktor SF und Wassergehalt EWC) verschiedener Hydrogele folgender Zusammensetzung: 100 mg α,ω-Diisocyanato-$PEG_x$ und 35 mg BSA in 2 ml 0.1 m Boratpufferlösung (pH 9.0). Die Bestimmung der Quellparameter erfolgte nach 72 h Inkubation in physiologischem Boratpuffer (0.1 m $H_3BO_3$, 1m $MgCl_2$, 1 m $CaCl_2$, 0.15 m NaCl; pH 7.0) bei RT und 24 h Trocknung bei 70 °C.

| m PEG [g/mol] | SF X | EWC [%] |
|---|---|---|
| 6000 | 29,3 | 96,7 |
| 10000 | 35,9 | 97,3 |
| 15000 | 43,4 | 97,7 |
| 20000 | 47,2 | 97,9 |
| 35000 | 63,4 | 98,5 |

Hierbei ist zu beobachten, daß eine stetige Zunahme der Wasseraufnahmekapazität in Richtung höherer eingesetzter Molekulargewichte von PEG vorliegt. Eine Wiederholung des Versuches nach durchgeführter Trocknung der Hydrogele (Dehydratisierung im Hochvakuum bis zur Gewichtskonstanz) zeigt eine Irreversibilität hinsichtlich der maximalen Wasseraufnahmekapazität auf. Tatsächlich liegen die Wassergehalte nach erneutem Quellen, in Anschluß an einen Trocknungsschritt, bei 60 - 70 % der vor dem Trocknen erreichbaren Wassergehalte.
Hinsichtlich der Sauerstoffdurchlässigkeit der vorliegenden Hydrogele kann angenommen werden, daß ab einem Wassergehalt > 94 % die Sauerstoffdiffusion innerhalb der Hydrogele der einer wäßrigen Lösung entspricht (Corkhill et al. 1990, Crit. Rev. Biocompatibility., 5, 363).

Methode zur Aktivitätsbestimmung von SOD

Für die Messung der Aktivität der verschiedenen SOD-Enzyme wurde der Biotech SOD-525-Assay (OXIS International S.A., Frankreich) eingesetzt. Dieser Assay basiert auf dem durch SOD verursachten Anstieg der Geschwindigkeitskonstante der Autooxidationsreaktion des Catechols 5,6,6a,11b-Tetrahydro-3,9,10-Trihydroxybenzo[c] fluoren (BXT-01050) in wäßriger alkalischer Lösung (Nebot et al., 1993, Analytical Biochemistry, 214, 442). Diese Autooxidationsreaktion erzeugt ein Chromophor mit einer maximalen Absorption bei 525 nm. Der Assay wird in einem luftgesättigten Puffer mit 50 mM 2-Amino-2-Methyl-1,3-Propandiol, 0.1 mM Diethylentriaminpentaessigsäure und 3 mM Borsäure, pH 8.8, bei 37 °C durchgeführt. Kinetiken werden bei 525 nm innerhalb einer Minute nach Zugabe von BXT-01050 gemessen. Die SOD-Aktivität wird bestimmt durch das Verhältnis der Geschwindigkeitskonstante der Autooxidationsreaktion $V_s/V_c$, die in Gegenwart ($V_s$) und in Abwesenheit ($V_c$) der Probe gemessen wird. Eine SOD-Einheit (U-525) wird definiert als die Aktivität, die den Hintergrund der Autooxidationsreaktion verdoppelt, das heißt $V_s/V_c = 2$.

Methode zur Aktivitätsbestimmung von Katalase

Katalase (EC 1.11.1.6, Boehringer Mannheim) katalysiert die Spaltung von Wasserstoffperoxid ($H_2O_2$) in Sauerstoff und Wasser. Die Aktivitätsbestimmung des gelösten Enzyms erfolgt mit einem spektrophotometrischen Assay

modifiziert nach R. F. Beers und I. W. Sizers (J. Biol. Chem. 195, 133 (1952)). Die Extinktionsabnahme bei 240 nm korreliert hierbei mit der Abnahme von Wasserstoffperoxid durch Katalase-Aktivität im Reaktionsgemisch.

In einer Quartzküvette (Hellma, Jena) werden 3 ml Phosphatpuffer (50 mM, pH 7.0) (Referenzposition) bzw. 3 ml eines mit $H_2O_2$ versetzten Phosphatpuffers (0,05 mM, pH 7) (Probe) mit je 0,05 ml der Katalase-haltigen Probe gegeben. Dabei wird die Wasserstoffperoxidkonzentration in dem $H_2O_2$-haltigen Puffer durch Extinktionsmessung bei 240 nm kontrolliert, deren Absorption bei 0,500 +/- 0,01 liegt. Nach Mischen der Komponenten in den Quartzküvetten (Schichtdicke 10 mm) wird die Reaktion solange beobachtet, bis die Extinktion einen Wert von 0,450 erreicht. Ab dann wird mit einer Stoppuhr diejenige Zeitspanne erfaßt, die benötigt wird, die Extinktion auf 0,400 zu vermindern. Die Enzymkonzentration in der Küvette wird dahingehend angepaßt, daß die hierfür benötigte Zeit 20 s +/- 2 s beträgt. Die Errechnung der Volumenaktivität erfolgt nach folgender Formel:

Volumenaktivität [U/ml] = (17 x 13) / gestoppte Zeit [s] x 0,05

Gesamtaktivität der Probe [U] = Vol. akt. [U/ml] x Verdünnung x Probenvolumen [ml]

Ein Unit ist dabei definiert als diejenige Enzymaktivität. die 1 µmol Wasserstoffperoxid unter den gewählten Assaybedingungen (25 °C, pH 7.0) pro Minute disproportioniert.

Methode zur Aktivitätsbestimmung von Collagenase

Collagenase (EC 3.4.24.3, Boehringer Mannheim) katalysiert die hydrolytische Spaltung von N-(3[2-Furyl] Acryolyl)-Leu-Gly-Pro-Ala (FALGPA) (Sigma Chemical Steinheim) an der Peptidbindung des Substrates zwischen den Aminosäuren Leucin und Glycin. Die Aktivitätsbestimmung des gelösten Enzyms erfolgt mit einem spektrophotometrischen Assay modifiziert nach H.E. van Wart und D.R. Steinbrink (Anal. Biochem. 113, 356 (1981)). Die Extinktionszunahme bei 345 nm korreliert hierbei mit der Freisetzung von gefärbtem N-(3-[2-Furyl]Acryloyl)-Leu aus dem Substrat durch Collagenase-Aktivität im Reaktionsgemisch.

In einer Quarzküvette (Schichtdicke 10mm, Hellma Jena) werden 2.9 ml Pufferlösung (50 mM Tricin, 10 mM Calciumchlorid, 400 mM Natriumchlorid, pH 7.5) mit 0.1 ml der collagenasehaltigen (2 units/ml) Probe (Probenposition) bzw. mit 0.1 ml destilliertem Wasser (Referenzposition) versetzt. Nach Mischen der Komponenten in den Quarzküvetten und Temperatureinstellung auf 25 °C wird die resultierende Extinktionsdifferenz über einen Zeitraum von fünf Minuten bei 345 nm kontrolliert. Die Berechnung der Volumenaktivität der Probe erfolgt nach folgender Formel:

Volumenaktivität der Probe [U/ml] =

DE nm/min Probe - DE nm/min Referenzposition / (0.53) x (mg Enzym / ml Reaktions

mix)

DE = Extinktionsdifferenz

Gesamtaktivität der Probe [U] = Vol.akt. [U/ml] x Verdünnung x Probenvolumen [ml]

Eine Unit ist definiert als diejenige Enzymaktivität, die 1.0 mmol FALGPA unter den gewählten Assaybedingungen (25 °C, pH 7.5) pro Minute hydrolysiert.

Methode zur Aktivitätsbestimmung von Trypsin

Trypsin (EC 3.4.21.4, Sigma Chemical Steinheim) katalysiert die Spaltung von Peptidbindungen am C-terminalen Ende der Aminosäuren Arginin und Lysin. Die Aktivitätsbestimmung des gelösten Enzyms erfolgt mit einem spektrophotometrischem Assay modifiziert nach Hummel (Can. J. Biochem. Physiol. 37, 1393 (1959)). Die Extinktionszunahme bei 257 nm korreliert mit der Freisetzung von *p*-Toluolsulfonyl-l-arginin aus *p*-Toluolsulfonyl-l-argininmethylester (Sigma Chemical Steinheim).

In einer Quarzküvette (Schichtdicke 10mm, Hellma Jena) werden 2.6 ml Pufferlösung (0.65 m Tris(hydroxymethyl)-aminomethan, 11 mM Calciumchlorid-Dihydrat, pH 8.1) und 0.3 ml Substratlösung (10 mM *p*-Toluolsulfonyl-l-arginin-

methylester) gegeben und mit 0.1 ml Enzymlösung (0.5 U in 1 ml 0.001 n Salzsäure) versetzt. Nach Mischen in der Quarzküvette und Temperatureinstellung auf 25 °C wird die Extinktionszunahme über einen Zeitraum von fünf Minuten gegen destilliertes Wasser bestimmt, wobei ein konstanter Wert pro Minute ermittelt wird. Die Berechnung der Volumenaktivität der Probe erfolgt nach folgender Formel:

Volumenaktivität der Probe [U/ml] =

DE nm/min Probe x Probevolumen / (0.54) x (mg Enzym / ml Reaktionsmix)

DE = Extinktionsdifferenz
Gesamtaktivität der Probe [U] = Vol.akt. [U/ml] x Verdünnung x Probenvolumen [ml]
Eine Unit ist definiert als diejenige Enzymaktivität, die die Bildung von 1 mmol *p*-Toluolsulfonyl-I-arginin unter den gewählten Assaybedingungen (25 °C, pH 7.5) pro Minute bewirkt.

Methode zur Aktivitätsbestimmung von Elastase

Elastase (EC 3.4.21.36, Sigma Chemical Steinheim) katalysiert die Spaltung von zum Beispiel Elastin in Aminosäuren und Peptide. Die Aktivitätsbestimmung des gelösten Enzyms erfolgt mit einem spektrophotometrischem Assay modifiziert nach Sacher et al. (Proc. Soc. Exp. Biol. Med 90, 1955 (1955)). Die Extinktionszunahme bei 590 nm korreliert mit der hydrolytischen Freisetzung von Orcein aus Elastin-orcein (Sigma Chemical Steinheim).
In einem Erlenmeyerkolben werden 20 mg Elastin-orcein eingewogen und mit 1.5 ml Tris(hydroxymethyl)-aminomethan-hydrochlorid (197 mM, pH 8.8) versetzt und auf 37 °C temperiert. Nach Zugabe von 0.5 ml Enzymlösung wird der Kolben verschlossen und bei 37 °C gelagert. Durch Zugabe von 2 ml Phosphatpufferlösung (500 mM, pH 6.0) wird die Reaktion nach 20 Minuten unterbrochen. Der Ansatz wird filtriert und der Kolben mit 1 ml destilliertem Wasser gespült. In einer Quarzküvette (Schichtdicke 10mm, Hellma Jena) wird die Extinktion von 3 ml Probenlösung gegen 3 ml einer Referenzposition (2.0 ml Tris(hydroxymethyl)-aminomethan-hydrochlorid (197 mM, pH 8.8), 2.0 ml Phosphatpufferlösung (500 mM, pH 6.0) und 1.0 ml destilliertes Wasser) bei 590 nm bestimmt. Die Berechnung der Volumenaktivität der Probe erfolgt nach folgender Formel:

Volumenaktivität der Probe [U/ml] =

$E_{590}$ x 19.45 - 0.1185 = mg gespaltenes Elastin-orcein

mg gespaltenes Elastin-orcein / (mg Enzym/0.5 ml Reaktionsmix)

Gesamtaktivität der Probe [U] = Vol.akt. [U/ml] x Verdünnung x Probenvolumen [ml]

Eine Unit ist definiert als diejenige Enzymaktivität, die innerhalb von 20 min 1 mg Elastin unter den gewählten Assaybedingungen (37 °C, pH 8.8) spaltet.
Im folgenden sollen anhand mehrerer Beispiele besonders vorteilhafte Ausführungsformen der Erfindung dargestellt werden, ohne damit die Erfindung unnötig einschränken zu wollen.

**Beispiel 1 - Herstellung von Hydrogelen mit Serumalbumin**

Die Synthese der Hydrogels erfolgt durch eine kovalente Vernetzung von $\alpha,\omega$-aktiviertem PEG mit Albumin aus Rinderserum. Zu einer 2 %-igen (m/V) Proteinlösung in 0.1 m Boratpuffer (pH 6.5 - 9.0) werden verschiedene Mengen (1 - 20 % m/V) aktiviertes PEG zugegeben und solange gerührt, bis der Gelpunkt einsetzt. Anschließend wird das gebildete Hydrogel 12 h bei RT gelagert, um eine vollständige Vemetzung zu erlangen. Um unvemetztes PEG und Protein abzutrennen, wird das Hydrogel in 15 ml physiologischem Boratpuffer (0.1 m $H_3BO_3$, 1m $MgCl_2$, 1 m $CaCl_2$, 0.15 m NaCl; pH 7.0) für 48 h inkubiert, wobei die Waschlösungen mehrmals gewechselt werden. Die dabei eluierten Mengen an Protein werden mithilfe des BCA- und Micro BCA-Nachweises (Rhoderick, Jarvis and Hyland, 1989, Anal. Biochem., 180, 136) und die eluierten Mengen an PEG mithilfe des Jod-Nachweises (Sims and Snape, 1980, Anal. Biochem.,107, 60) evaluiert.
Analog findet die Vernetzungsreaktion in 0.05 M Natriumbicarbonatpuffer mit den pH-Werten 8.5, 9.0 und 9.3 statt sowie in einem 0.1 M Natriumveronalpuffer bei pH 9.0. Die in Natriumphosphatpuffer hergestellten Hydrogele können

durch Austausch in physiologischen Boratpuffer stabil gehalten werden.

Die folgende Darstellung zeigt schematisch die Vernetzung von $PEG_{35000}$ mit Rinderserumalbumin (BSA).

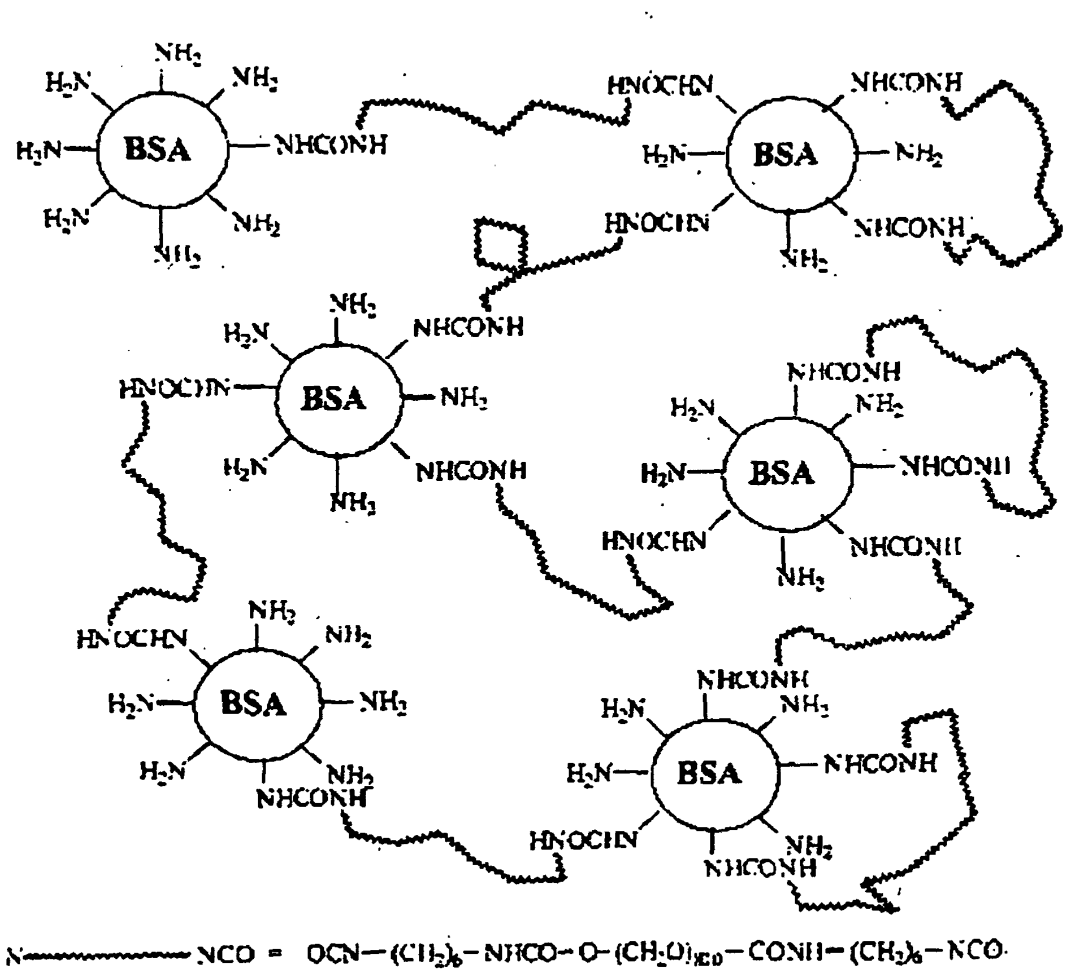

.**Beispiel 2 - Herstellung von Hydrogelen mit Superoxiddismutase**

Das ubiquitäre Enzym SOD tritt als dimere oder tetramere Form auf. Die verschiedenen Enzyme mit Molekulargewichten von 32.000 bis 56.000 Da haben Metall-Ionen wie Kupfer und Zink (Cu-Zn-SOD), Mangan (Mn-SOD) oder Eisen (Fe-SOD) als Co-Faktoren im katalytischen Zentrum.

Die Synthese der Hydrogele erfolgt durch eine kovalente Vernetzung von $\alpha,\omega$-aktiviertem PEG mit Superoxiddismutase aus Rind und Hefe. Zu einer 2 %-igen (m/V) Proteinlösung in 0.1 m Boratpuffer (pH 9.0) werden verschieden Mengen (6 - 25 % m/V) aktiviertes PEG zugegeben und solange gerührt, bis der Gelpunkt einsetzt. Es werden grünlich transparente Gele erhalten.

Anschließend werden die Hydrogele 12 h bei RT gelagert, um eine vollständige Vernetzung zu erlangen. Um unvemetztes PEG und Protein abzutrennen, werden die Hydrogele in 15 ml physiologischem Boratpuffer (0.1 m $H_3BO_3$, 0.1 m $MgCl_2$, 0.1 m $CaCl_2$, 0.15 m NaCl; pH 7.0) für 48 h inkubiert, wobei die Waschlösungen mehrmals gewechselt werden. Eine Gelbildung wurde für die PEG $PEG_{20000}$, $PEG_{15000}$, $PEG_{10000}$ und $PEG_{6000}$ beobachtet:.

**Beispiel 3 - Herstellung von Hydrogelen mit Katalase**

Die weitverbreitetste Form der Katalase ist ein Homotetramer (235.000 Da, Rinderleber) mit einer porphyrinischen Gruppe mit einem Eisenatom je Untereinheit.

Die Synthese der Hydrogele erfolgt durch eine kovalente Vernetzung von $\alpha,\omega$-viertem PEG mit Katalase aus Rinderleber. Zu einer 2.5 %-igen (m/V)Proteinlösung in 0.1 m Boratpuffer (pH 9.0) werden 7.5 % (m/V) aktiviertes $PEG_{10000}$ bzw. 15 % (m/V) aktiviertes $PEG_{6000}$ zugegeben und solange gerührt, bis der Gelpunkt einsetzt. Es werden bräunlich transparente Gele erhalten.
Anschließend werden die Hydrogele 12 h bei RT gelagert, um eine vollständige Vernetzung zu erlangen. Um unvemetztes PEG und Protein abzutrennen, werden die Hydrogele in 15 ml physiologischem Boratpuffer (0.1 m $H_3BO_3$, 0.1 m $MgCl_2$, 0.1 m $CaCl_2$, 0.15 m NaCl; pH 7.0) für 48 h inkubiert, wobei die Waschlösungen mehrmals gewechselt werden.

**Beispiel 4 - Herstellung von Hydrogelen mit Superoxiddismutase und Katalase**

Die Synthese der Hydrogele erfolgt durch eine kovalente Vernetzung von $\alpha,\omega$-aktiviertem PEG mit Superoxiddismutase aus Hefe und Katalase aus Rinderleber. Zu einer 2.4 %-igen (m/V) Superoxiddismutase-Lösung in 0.1 m Boratpuffer (pH 9.0) werden 0.8 - 0.15 % (m/V) Katalase und 5 % (m/V) aktiviertes PEG zugegeben und solange gerührt, bis der Gelpunkt einsetzt. Es werden grün-bräunlich transparente Gele erhalten.
Anschließend werden die Hydrogele 12 h bei RT gelagert, um eine vollständige Vernetzung zu erlangen. Um unvemetztes PEG und Protein abzutrennen, werden die Hydrogele in 15 ml physiologischem Boratpuffer (0.1 m $H_3BO_3$, 0.1 m $MgCl_2$, 0.1 m $CaCl_2$, 0.15 m NaCl; pH 7.0) für 48 h inkubiert, wobei die Waschlösungen mehrmals gewechselt werden.
Eine Gelbildung wurde für $PEG_{10000}$ und $PEG_{6000}$ beobachtet.

**Beispiel 5 - Herstellung von Hydrogelen mit Collagenase**

Die Synthese der Hydrogele erfolgt durch eine kovalente Vernetzung von $\alpha,\omega$-aktiviertem PEG mit Collagenase aus Achromobacter iophagus. Zu einer 5 %-igen (m/V) Proteinlösung in 0.1 m Boratpuffer (pH 9.0) werden 7.5 - 15 % (m/V) aktiviertes $PEG_{15000}$ bzw. aktiviertes $PEG_{10000}$ zugegeben und solange gerührt, bis der Gelpunkt einsetzt. Es werden rot-bräunlich transparente Gele erhalten.
Anschließend werden die Hydrogele 12 h bei RT gelagert, um eine vollständige Vernetzung zu erlangen. Um unvernetztes PEG und Protein abzutrennen, werden die Hydrogele in 15 ml physiologischem Boratpuffer (0.1 m $H_3BO_3$, 0.1 m $MgCl_2$, 0.1 m $CaCl_2$, 0.15 m NaCl; pH 7.0) für 48 h inkubiert, wobei die Waschlösungen mehrmals gewechselt werden.

**Beispiel 6 - Herstellung von Hydrogelen mit Lysozym**

Die Synthese der Hydrogele erfolgt durch eine kovalente Vernetzung von $\alpha,\omega$-aktiviertem PEG mit Lysozym aus Hühnereiweiß. Zu einer 2.5 - 5 %-igen (m/V) Proteinlösung in 0.1 m Boratpuffer (pH 9.0) werden 7.5 - 15 % (m/V) aktiviertes PEG zugegeben und solange gerührt, bis der Gelpunkt einsetzt. Es werden milchig-trübe Gele erhalten.
Anschließend werden die Hydrogele 12 h bei RT gelagert, um eine vollständige Vernetzung zu erlangen. Um unvernetztes PEG und Protein abzutrennen, wird das Hydrogel in 15 ml physiologischem Boratpuffer (0.1 m $H_3BO_3$, 0.1 m $MgCl_2$, 0.1 m $CaCl_2$, 0.15 m NaCl; pH 7.0) für 48 h inkubiert, wobei die Waschlösungen mehrmals gewechselt werden.
Eine Gelbildung konnte für folgende PEG beobachtet werden: $PEG_{35000}$, $PEG_{20000}$, $PEG_{15000}$, $PEG_{10000}$ und $PEG_{6000}$.

**Beispiel 7 - Herstellung von Hydrogelen mit Trypsin**

Die Synthese der Hydrogele erfolgte durch eine kovalente Vernetzung von $\alpha,\omega$ - aktiviertem PEG mit Trypsin aus Rind. Zu einer 2.5 - 5 %-igen (m/V) Proteinlösung in 0.1 m Boratpuffer (pH 9.0) wurden 7.5 - 15 % (m/V) aktiviertes PEG zugegeben und solange gerührt, bis der Gelpunkt einsetzte. Es wurden milchig-trübe Gele erhalten. Anschließend werden die Hydrogele 12 h bei RT gelagert, um eine vollständige Vernetzung zu erlangen. Um unvernetztes PEG und Protein abzutrennen, wurde das Hydrogel in 15 ml physiologischem Boratpuffer (0.1 m $H_3BO_3$, 0.1 m $MgCl_2$, 0.1 m $CaCl_2$, 0.15 m NaCl; pH 7.0) für 48 h inkubiert, wobei die Waschlösungen mehrmals gewechselt wurden. Eine Gelbildung konnte für folgende PEG beobachtet werden: $PEG_{10000}$ und $PEG_{6000}$.

**Beispiel 8 - Herstellung von Hydrogelen mit Phosphatase**

Die Synthese der Hydrogele erfolgte durch eine kovalente Vernetzung von $\alpha,\omega$ - aktiviertem PEG mit Phosphatase aus Weizenkeimen. Zu einer 2.5 %-igen (m/V) Proteinlösung in 0.1 m Boratpuffer (pH 9.0) wurden 10 - 15 % (m/V) aktiviertes PEG zugegeben und solange gerührt, bis der Gelpunkt einsetzte. Es wurden bräunliche Gele erhalten.
Anschließend werden die Hydrogele 12 h bei RT gelagert, um eine vollständige Vernetzung zu erlangen. Um unvemetztes PEG und Protein abzutrennen, wurde das Hydrogel in 15 ml physiologischem Boratpuffer (0.1 m $H_3BO_3$, 0.1 m $MgCl_2$, 0.1 m $CaCl_2$, 0.15 m NaCl; pH 7.0) für 48 h inkubiert, wobei die Waschlösungen mehrmals gewechselt wurden.
Eine Gelbildung konnte für folgende PEG beobachtet werden: $PEG_{10000}$ und $PEG_{6000}$.

**Patentansprüche**

1. Hydrogel, bestehend aus zumindest einem Protein und/oder Enzym und/oder SOD/Katalase-Enzym-Mimic u nd PEGs s owie T eilen von P roteinen u nd E nzymen u nd/ode r ekombinant hergestellten Proteinen und Enzymen, wobei die Verbindung zwischen den Proteinen und/oder Enzymen und den PEGs über Harnstoffgruppen erfolgt, dadurch gekenntzeichnet, dass zur Aktivierung des PEGs aliphatische Diisocyanate, insbesondere 1,6-Hexamethylen-diisocyanat, eingesetzt werden.

2. Hydrogel nach Anspruch 1, **dadurch gekennzeichnet, daß** das PEG ein Molgewicht von 8.000 bis 18.000 g/mol, insbesondere 10,000 bis 15.000 g/mol aufweist.

3. Hydrogel nach den Ansprüchen 1 bis 2, **dadurch gekennzeichnet, daß** als Proteine Antikörper, Matrix-Metallo-Proteasen, Enzyminhibitoren, Peptide verwendet werden.

4. Hydrogel nach den Ansprüchen 1 bis 3, **dadurch gekennzeichnet, daß** als Proteine Radikalfänger wie Superoxiddismutase, Katalase, Glutathion-Peroxidase, Myeloperoxidase und SOD/Katalase-Enzym-Mimics und/oder Kombinationen daraus verwendet werden.

5. Hydrogel nach den Ansprüchen 1 bis 4, **dadurch gekennzeichnet, daß** als Proteine antimikrobiell wirksame Enzyme wie Lysozym und Hydrolasen eingesetzt werden.

6. Hydrogel nach den Ansprüchen 1 bis 5, **dadurch gekennzeichnet, daß** als Proteine phosphorylierend wirksame Enzyme wie Phosphatasen und Kinasen eingesetzt werden.

7. Hydrogel nach den Ansprüchen 1 bis 6, **dadurch gekennzeichnet, daß** als Proteine Wachstumsfaktoren wie PDGF eingesetzt werden.

8. Hydrogel nach den Ansprüchen 1 bis 7, **dadurch gekennzeichnet, daß** als Proteine proteolytisch wirkende Enzyme wie Collagenase, Trypsin, Elastase und/oder Kombinationen daraus eingesetzt werden.

9. Hydrogel nach den Ansprüchen 1 bis 8, **dadurch gekennzeichnet, daß** als Proteine proteinogene Protease-Inhibitoren wie Aprotinin, Sojabohnen-Trypsininhibitor und Alpha-2-Makroglobulin und/oder Kombinationen daraus eingesetzt werden.

10. Hydrogel nach den Ansprüchen 1 bis 9, **dadurch gekennzeichnet, daß** Proteine in Mischungen eingesetzt werde.

11. Verfahren zur Herstellung eines Hydrogels nach zumindest einem der vorherigen Ansprüche, **dadurch gekennzeichnet, dass**

    a) wasserfreie PEGs in Lösungsmittel mit aliphatischen Diisocyanat, insbesondere 1,6-Hexamethylen-diisocyanat, gegebenenfalls unter Zusatz eines Katalysators umgesetzt werden,
    b) das resultierende Produkt aus aktiviertem PEGs, insbesondere $\alpha,\omega$ - aktiviertem PEG, vom Lösungsmittel durch Abfiltern, Waschen oder Trocknung befreit wird,
    c) die aktivierten PEGs in wässriger Lösung mit Proteinen umgesetzt werden, wobei sich die Proteine in einem Puffer befinden, der bevorzugt so gewählt ist, dass die Proteine ihre biologische Aktivität erhalten,
    d) gegebenenfalls Reinigungsschritte und Waschungen vorgenommen werden.

12. Verfahren zur Herstellung eines Hydrogels nach Anspruch 11, **dadurch gekennzeichnet, daß** das Hydrogel dehydratisiert ist.

13. Hydrogel gemäß einem der vorherigen Ansprüche 1 bis 10 als Wundauflage.

14. Hydrogel gemäß einem der vorherigen Ansprüche 1 bis 10 als Auflage auf gegebenenfalls luft- und wasserdampfdurchlässigen Trägermaterialien wie Binden, Kompressen, Pflastern, Folien und/oder Filmen.

## Claims

1. Hydrogel comprising at least one protein and/or enzyme and/or SOD/catalase enzyme mimic and PEGs, and parts of proteins and enzymes and/or recombinant proteins and enzymes, the proteins and/or enzymes being connected to the PEGs via urea groups, **characterized in that** aliphatic diisocyanates, in particular 1,6-hexamethylene diisocyanate, are employed to activate the PEG.

2. Hydrogel according to Claim 1, **characterized in that** the PEG has a molecular weight of from 8.000 to 18.000 g/mol, in particular from 10,000 to 15,000 g/mol.

3. Hydrogel according to Claims 1 to 2, **characterized in that** antibodies, matrix metalloproteases, enzyme inhibitors, peptides are used as proteins.

4. Hydrogel according to Claims 1 to 3, **characterized in that** free radical scavengers such as superoxide dismutase, catalase, glutathione peroxidase, myeloperoxidase and SOD/catalase enzyme mimics and/or combinations thereof are used as proteins.

5. Hydrogel according to Claims 1 to 4, **characterized in that** enzymes with antimicrobial activity, such as lysozyme and hydrolases, are employed as proteins.

6. Hydrogel according to Claims 1 to 5, **characterized in that** enzymes with phosphorylating activity, such as phosphatases and kinases, are employed as proteins.

7. Hydrogel according to Claims 1 to 6, **characterized in that** growth factors such as PDGF are employed as proteins.

8. Hydrogel according to Claims 1 to 7, **characterized in that** enzymes with proteolytic activity, such as collagenase, trypsin, elastase and/or combinations thereof, are employed as proteins.

9. Hydrogel according to Claims 1 to 8, **characterized in that** proteinogenic protease inhibitors such as aprotinin, soya bean trypsin inhibitor and alpha-2-macroglobulin and/or combinations thereof are employed as proteins.

10. Hydrogel according to Claims 1 to 9, **characterized in that** proteins are employed in mixtures.

11. Process for producing a hydrogel according to at least one of the preceding claims, **characterized in that**

    a) anhydrous PEGs are reacted with aliphatic diisocyanate, in particular 1,6-hexamethylene diisocyanate, in a solvent, where appropriate with the addition of a catalyst,
    b) the solvent is removed from the resulting product of activated PEGs, in particular $\alpha,\omega$-activated PEG, by filtration, washing or drying,
    c) the activated PEGs are reacted in aqueous solution with proteins, the proteins being present in a buffer which is preferably chosen so that the proteins retain their biological activity,
    d) where appropriate, purification steps and washes are carried out.

12. Process for producing a hydrogel according to Claim 11, **characterized in that** the hydrogel is dehydrated.

13. Hydrogel according to one of the preceding Claims 1 to 10 as wound dressing.

14. Hydrogel according to one of the preceding claims 1 to 10 for application to substrate materials which are permeable to air and water vapour, where appropriate, such as bandages, compresses, plasters, sheets and/or films.

## Revendications

1. Hydrogel, constitué par au moins une protéine et/ou une enzyme et/ou une enzyme mimique de SOD/catalase et des PEG ainsi que des parties de protéines et d'enzymes et/ou des protéines préparées par recombinaison et des enzymes, la liaison entre les protéines et/ou les enzymes et les PEG étant réalisée via des groupes urée, **caractérisé en ce qu'**on utilise pour l'activation du PEG des diisocyanates aliphatiques, en particulier le 1,6-hexaméthylènediisocyanate.

2. Hydrogel selon la revendication 1, **caractérisé en ce que** le PEG présente un poids moléculaire de 8000 à 18000 g/mole, en particulier de 10000 à 15000 g/mole.

3. Hydrogel selon les revendications 1 à 2, **caractérisé en ce qu'**on utilise comme protéines des anticorps, des métalloprotéases de matrice, des inhibiteurs d'enzymes, des peptides.

4. Hydrogel selon les revendications 1 à 3, **caractérisé en ce qu'**on utilise comme protéines des pièges de radicaux tels que la superoxyde-dismutase, la catalase, la glutathion-peroxydase, la myéloperoxydase et des enzymes mimiques de SOD/catalase et/ou des combinaisons de celles-ci.

5. Hydrogel selon les revendications 1 à 4, **caractérisé en ce qu'**on utilise comme protéines des enzymes à effet antimicrobien telles que le lysozyme et les hydrolases.

6. Hydrogel selon les revendications 1 à 5, **caractérisé en ce qu'**on utilise comme protéines des enzymes à activité phosphorylante telles que les phosphatases et les kinases.

7. Hydrogel selon les revendications 1 à 6, **caractérisé en ce qu'**on utilise comme protéines des facteurs de croissance tels que le PDGF.

8. Hydrogel selon les revendications 1 à 7, **caractérisé en ce qu'**on utilise comme protéines des enzymes à effet protéolytique telles que la collagénase, la trypsine, l'élastase et/ou des combinaisons de celles-ci.

9. Hydrogel selon les revendications 1 à 8, **caractérisé en ce qu'**on utilise comme protéines des inhibiteurs protéinogènes de protéase, tels que l'aprotinine, l'inhibiteur de trypsine de fèves de soja et l'alpha-2-macroglobuline et/ou des combinaisons de ceux-ci.

10. Hydrogel selon les revendications 1 à 9, **caractérisé en ce que** les protéines sont utilisées dans des mélanges.

11. Procédé pour la préparation d'un hydrogel selon au moins l'une quelconque des revendications précédentes, **caractérisé en ce que**

    a) on transforme des PEG anhydres dans un solvant avec un diisocyanate aliphatique, en particulier le 1,6-hexaméthylènediisocyanate, le cas échéant avec addition d'un catalyseur,
    b) on libère le produit obtenu, constitué par les PEG activés, en particulier le PEG $\alpha,\omega$-activé, du solvant par filtration, lavage ou séchage,
    c) on transforme les PEG activés en solution aqueuse avec des protéines, les protéines se trouvant dans un tampon qui est de préférence choisi de telle manière que les protéines conservent leur activité biologique,
    d) on réalise le cas échéant des étapes de nettoyage et des lavages.

12. Procédé pour la préparation d'un hydrogel selon la revendication 11, **caractérisé en ce que** l'hydrogel est déshydraté.

13. Hydrogel selon l'une quelconque des revendications précédentes 1 à 10 comme emplâtre pour plaies.

14. Hydrogel selon l'une quelconque des revendications précédentes 1 à 10 comme emplâtre sur des matériaux support le cas échéant perméables à l'air et à la vapeur d'eau, tels que des bandages, des compresses, des pansements, des feuilles et/ou des films.

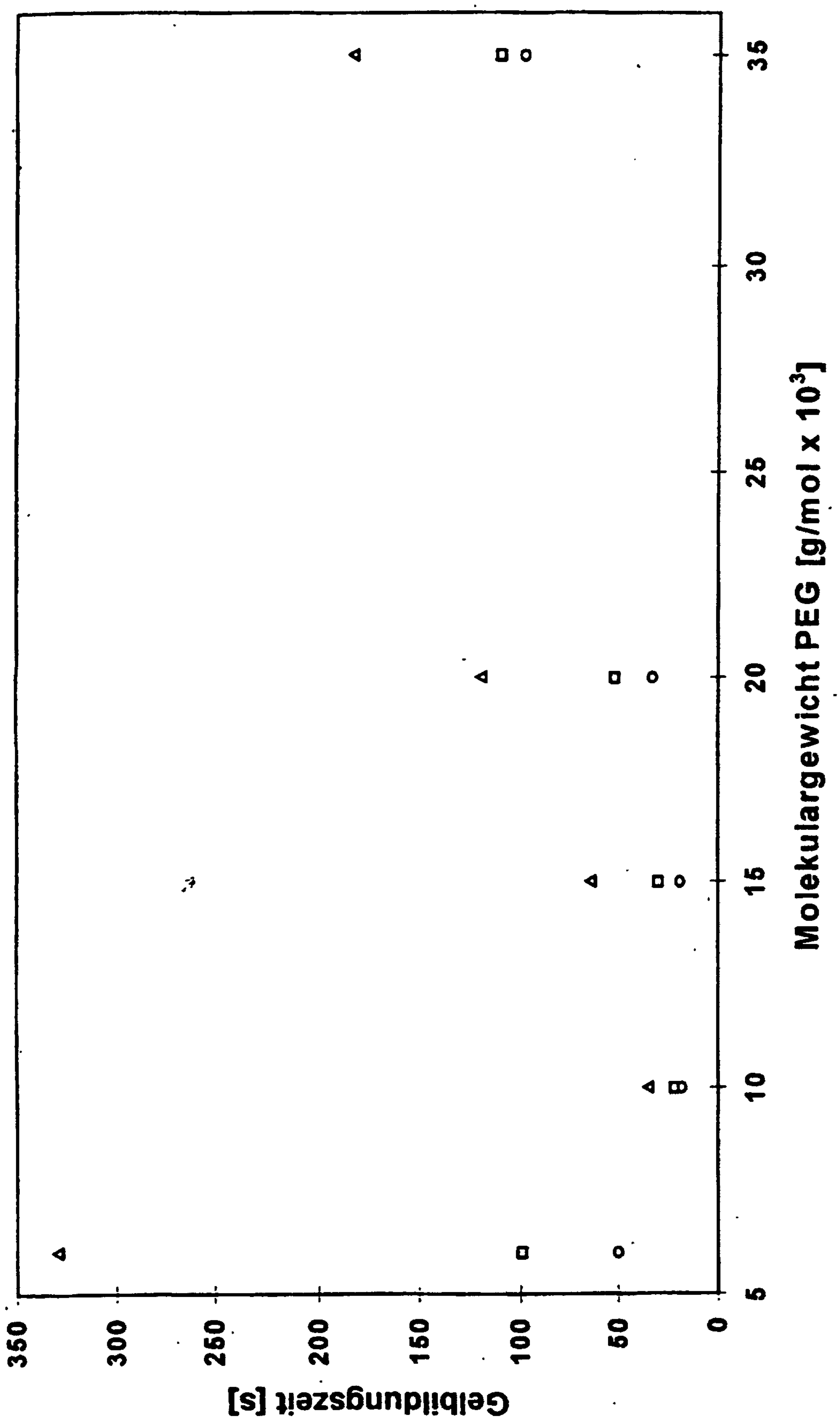
Gelbildungszeit [s]
0
50
100
150
200
250
300
350
5
10
15
20
25
30
35
Molekulargewicht PEG [g/mol x 10³]

Figur 1

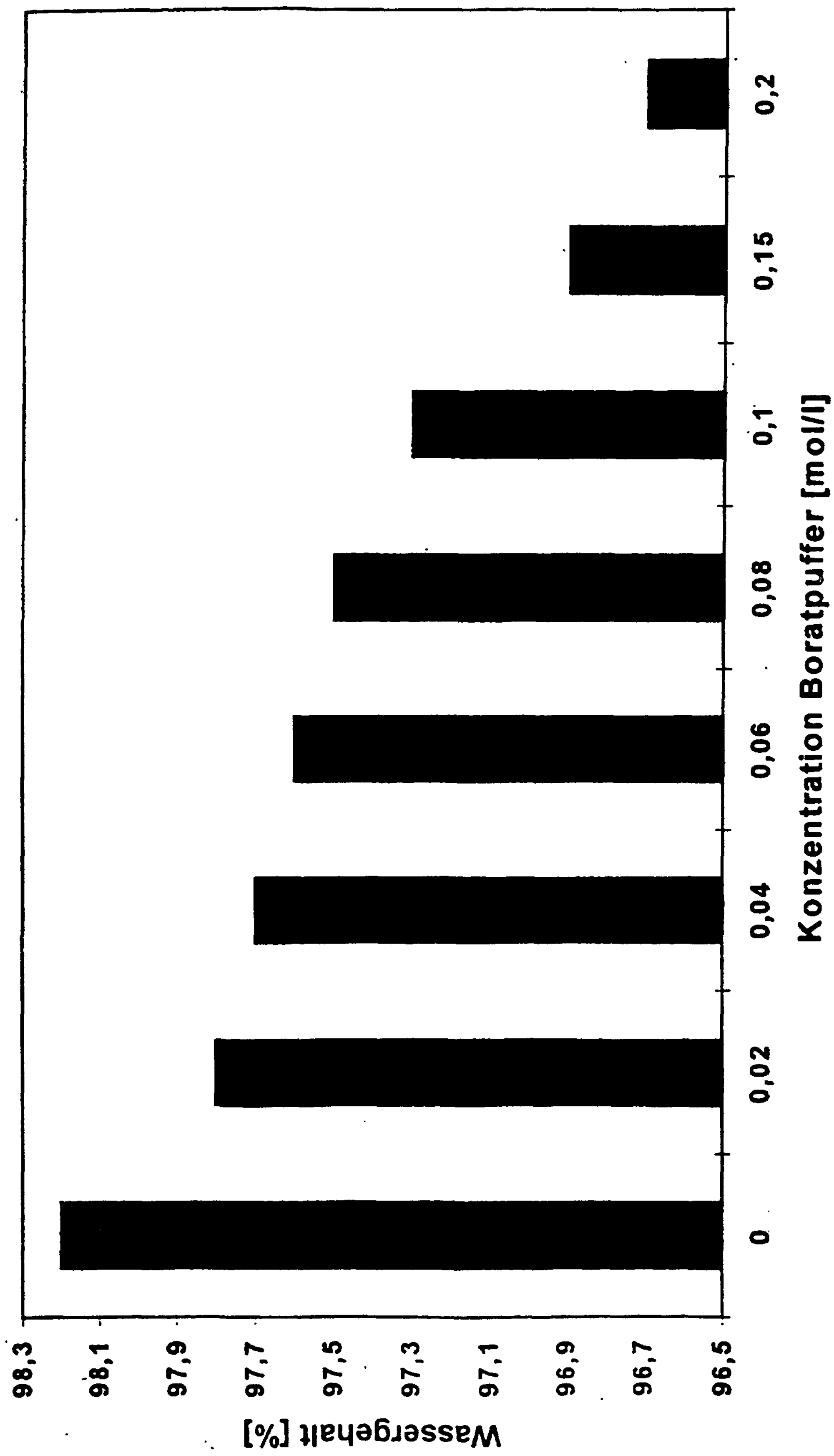
Wassergehalt [%]
98,3
98,1
97,9
97,7
97,5
97,3
97,1
96,9
96,7
96,5
0
0,02
0,04
0,06
0,08
0,1
0,15
0,2
Konzentration Boratpuffer [mol/l]

Figur 2